# EUROPÄISCHE PATENTSCHRIFT

(11) **EP 1 294 708 B1**
(45) Veröffentlichungstag und Bekanntmachung des Hinweises auf die Patenterteilung: **09.07.2014**
(21) Anmeldenummer: 01960363.8
(22) Anmeldetag: 22.06.2001
(51) Int. Cl.: C07D 401/14, C07D 401/04, C09B 25/00, C09D 7/00, C09D 11/02, C09D 11/00, C09B 67/22, C08K 5/3437, G03G 9/09

(54) **KRISTALLISATIONSMODIFIKATOREN AUF DER BASIS VON CHINOPHTHALONDERIVATEN**
QUINOPHTHALONE-DERIVATIVE BASED CRYSTALLIZATION MODIFIERS
MODIFICATEURS DE CRISTALLISATION A BASE DE DERIVES DE QUINOPHTHALONE

(30) Priorität: 29.06.2000 DE 10030780
(43) Veröffentlichungstag der Anmeldung: 26.03.2003
(73) Patentinhaber: BASF SE, 67056 Ludwigshafen (DE)
(72) Erfinder: HE, Jianing, 67069 Ludwigshafen (DE); SCHRÖCK, Manfred, 67134 Birkenheide (DE)
(86) Internationale Anmeldenummer: PCT/EP2001/007128
(87) Internationale Veröffentlichungsnummer: WO 2002/000643

(56) Entgegenhaltungen:
- EP-A- 0 335 237
- WO-A-01/27206
- WO-A-97/46623
- WO-A-98/32802
- DE-A- 1 770 960
- DE-A- 2 626 271
- DE-A- 2 638 528
- DE-A- 2 706 872

## Beschreibung

Die vorliegende Erfindung betrifft Chinophthalonderivate der allgemeinen Formel I 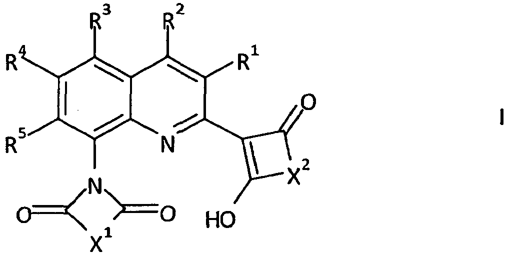 in der die Variablen folgende Bedeutung haben:
- R¹, R², R³ und R⁵: unabhängig voneinander Wasserstoff, Halogen oder C₁-C₄-Alkyl;
- R⁴: -SO₃H, -SO₃⁻ N⁺R⁶R⁷R⁸R⁹, -SO₂NR⁶R⁷, -CH₂R¹⁰, -COOH, -COO⁻ N⁺R⁶R⁷R⁸R⁹, -COOR¹¹ oder -NO₂;
- R⁶, R⁷, R⁸ und R⁹: unabhängig voneinander Wasserstoff; C₁-C₂₂-Alkyl oder C₂-C₂₂-Alkenyl, dessen Kohlenstoffkette jeweils durch eine oder mehrere Gruppierungen -O-, -S-, -NR¹²-, -CO- oder -SO₂-unterbrochen sein kann und/oder das ein- oder mehrfach durch Hydroxy, Halogen, Aryl, C₁-C₄-Alkoxy und/oder Acetyl substituiert sein kann; C₃-C₈-Cycloalkyl, dessen Kohlenstoffgerüst durch eine oder mehrere Gruppierungen -O-, -S-, -NR¹²- oder -CO- unterbrochen sein kann und/oder das ein- oder mehrfach durch Hydroxy, Halogen, Aryl, C₁-C₄-Alkoxy und/oder Acetyl substituiert sein kann; Dehydroabietyl oder Aryl; R⁶ und R⁷ bzw. R⁶, R⁷ und R⁸ zusammen einen das Stickstoffatom enthaltenden, 5- bis 7-gliedrigen cyclischen Rest, der weitere Heteroatome enthalten kann;
- R¹⁰: einen Rest 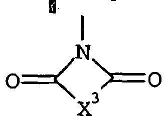
- R¹¹: einen der Alkylreste R⁶;
- R¹²: Wasserstoff oder C₁-C₄-Alkyl;
- X¹, X² und X³: unabhängig voneinander Arylen, das durch Halogen, Arylsulfonyl oder -COR¹³ oder -CO-C₆H₄-CO- substituiert sein kann;
- R¹³: C₁-C₃-Alkyl oder Phenyl,
und deren Verwendung als Kristallisationsmodifikatoren für organische Pigmente.

Außerdem betrifft die Erfindung ein Verfahren zur Überführung von Chinophthalonrohpigmenten in eine feinteilige Pigmentform.

Schließlich betrifft die Erfindung Pigmentzubereitungen, enthaltend

### A) mindestens ein Chinophthalonpigment der allgmeinen Formel II

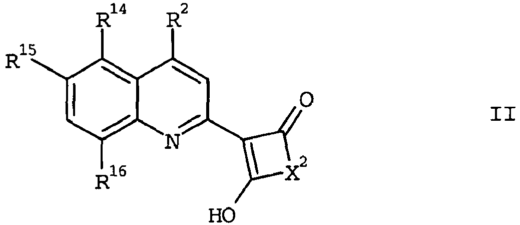

in der die Variablen folgende Bedeutung haben:
- R²: Wasserstoff, Halogen oder C₁-C₄-Alkyl;
einer der Reste R¹⁴, R¹⁵ und R¹⁶ einen Rest 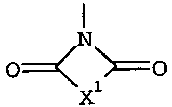 und die anderen Reste R¹⁴ bis R¹⁶ Wasserstoff;
- X¹ und X²: unabhängig voneinander Arylen, das durch Halogen, Arylsulfonyl oder -COR¹³ oder -CO-C₆H₄-CO- substituiert sein kann;
- R¹³: C₁-C₃-Alkyl oder Phenyl,
und

### B) mindestens ein Chinophthalonderivat I,

und die Verwendung der erfindungsgemäß erhaltenen Chinophthalonpigmente und der Pigmentzubereitungen zum Einfärben von Kunststoffen, Lacken, Druckfarben, Ink-Jet-Tinten, Farbfiltern und elektrophotographischen Tonern.

Chinophthalonpigmente stellen aufgrund ihrer Stabilität, insbesondere ihrer hohen Licht- und Wetterechtheit sowie Hitzebeständigkeit, interessante Gelbpigmente dar. Als besonders wichtiger Vertreter dieser Pigmentklasse sei C.I. Pigment Yellow 138 genannt (vgl. DE-A-17 70 960). Weitere Chinophthalonpigmente sind in den DE-A-26 26 271, 26 38 528 und 27 06 872 beschrieben.

Bei der Synthese fallen die Chinophthalonpigmente in grobkristalliner Form mit sehr heterogener Teilchengrößenverteilung an. Zur Überführung in eine für die Anwendung geeignete, koloristisch wertvolle Pigmentform werden diese Rohpigmente daher üblicherweise einer Formierung unterzogen.

Aus der DE-A-23 57 077 ist für diesen Zweck eine Mahlung des Rohpigments und anschließende Rekristallisation des Mahlgutes in einem organischen Lösungsmittel bekannt, und in der DE-A-27 46 164 wird eine Naßmahlung in wäßriger Suspension in schnellaufenden Rührwerkskugelmühlen durchgeführt.

Mit den beschriebenen Methoden wird zwar, zum Teil unter hohem Zeitwand, eine Teilchenzerkleinerung und damit eine Verbesserung der koloristischen Eigenschaften der Pigmente erreicht, jedoch läßt sich die Teilchengröße der erhaltenen Pigmente nur schwer steuern, und die Pigmente weisen in der Regel eine für eine Reihe von Anwendungen, z.B. als Farbmittel in Ink-Jet-Tinten, Farbfiltern und elektrophotographischen Tonern, unzureichende, da zu breite Teilchengrößenverteilung auf.

In der EP-A-335 237 wird die Verwendung von löslichen kationischen Farbstoffen auf Basis von Chinophthalon, die Imidazolylmethylreste tragen, beschrieben.

Aus der WO-A-97/46623 sind Ink-Jet-Tinten bekannt, die Chinophthalon-Dispersionsfarbstoffe als Farbmittel enthalten, die keine Phthalimidylreste aufweisen.

In der WO-A-98/32803 wird die Solubilisierung unlöslicher Chromophore durch den Einbau funktionalisierter Esterreste beschrieben. Nach Einarbeitung des Chromophors in das Anwendungsmedium werden diese Reste durch Wärmebehandlung wieder abgespalten.

Schließlich werden in der EP-A-1 138 723 (Stand der Technik nach Art. 54(3) EPÜ) Chinophthalonverbindungen offenbart, die keine Phthalimidylreste tragen und im Chinaldingerüst durch eine Hydroxylgruppe funktionalisiert sind und sich daher strukturell von den Chinophthalonderivaten I unterscheiden.

Der Erfindung lag daher die Aufgabe zugrunde, den genannten Nachteilen abzuhelfen und Chinophthalonpigmente mit vorteilhaften Anwendungseigenschaften, insbesondere auch mit vorteilhaften Teilchengrößenverteilungen, bereitzustellen.

Demgemäß wurden die Chinophthalonderivate der eingangs definierten Formel I und ihre Verwendung als Kristallisationsmodifikatoren für organische Pigmente gefunden.

Bevorzugte Chinophthalonderivate I sind dem Unteranspruch zu entnehmen.

Außerdem wurde ein Verfahren zur Überführung von Chinophthalonrohpigmenten in eine feinteilige Pigmentform gefunden, welches dadurch gekennzeichnet ist, daß man die Formierung des Chinophthalonrohpigments in Gegenwart der Chinophthalonderivate I vornimmt.

Schließlich wurden Pigmentzubereitungen gefunden, welche
A) mindestens ein Chinophthalonpigment II und
B) mindestens ein Chinophthalonderivat I enthalten, und die Verwendung der erfindungsgemäß erhaltenen Chinophthalonpigmente und der Pigmentzubereitungen zum Einfärben von Kunststoffen, Lacken, Druckfarben, Ink-Jet-Tinten, Farbfiltern und elektrophotographischen Tonern gefunden.

Alle in Formel I und den nachfolgend beschriebenen Formeln I, II und III auftretenden Alkylketten und Alkenylketten können geradkettig oder verzweigt sein.

Als Beispiele für Alkylreste seien im einzelnen beispielhaft genannt: Methyl, Ethyl, Propyl, Isopropyl, Butyl, Isobutyl, sec.-Butyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen).

Als Beispiele für Alkylreste, deren Kohlenstoffkette durch eine oder mehrere Gruppierungen -O-, -S-, -NR¹²-, -CO- oder -SO₂- unterbrochen sein kann, bzw. für alkoxy- und alkanoylsubstituierte Alkylreste seien folgende Reste aufgeführt:
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2-und 3-Propoxypropyl, 2- und 3- Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3- Butylthiopropyl, 2- und 4-Methylthiobutyl, 2- und 4-Ethylthiobutyl, 2-und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,22-Tetrathiatetradecyl;
2-Monomethyl- und 2-Monoethylaminoethyl, 2-Dimethylaminoethyl, 2-und 3-Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Buthylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl.

Als Beispiele für C₂-C₂₂-Alkenylreste seien beispielhaft Oleyl, Linoleyl und Linolenyl genannt.

Beispiele für Alkoxyreste sind Methoxy, Ethoxy, Propoxy, Isopropoxy, Butoxy, Isobutoxy, sec.-Butoxy und tert.-Butoxy.

Als Cycloalkylreste seien beispielhaft Cyclopropyl, Cyclobutyl, Cyclopentyl, Cyclohexyl, Cycloheptyl und Cyclooctyl aufgeführt. Heteroatome enthaltende Cycloalkylreste sind z.B. Dioxanyl, Morpholinyl, Tetrahydrofuryl, Pyrrolidinyl und Piperidinyl.

Arylreste sind beispielsweise Phenyl und 1- und 2-Naphthyl.

Halogen bedeutet insbesondere Chlor oder Brom, wobei Chlor bevorzugt ist.

Substituierte Alkylreste weisen vorzugsweise eine Kette mit bis zu 6 C-Atomen auf und tragen bevorzugt einen oder zwei Substituenten. Beispiele sind: 2-Hydroxyethyl, 2- und 3-Hydroxypropyl, 1-Hydroxyprop-2-yl und 2- und 4-Hydroxybutyl und Benzyl.

Als Beispiele für das Stickstoffatom enthaltende, aus R⁶ und R⁷ bzw. R⁶ bis R⁸ gebildete 5- bis 7-gliedrige cyclische Reste, die benzanelliert sein können, seien genannt: Morpholinyl, Pyrrolidi - nyl, Piperidyl, Pyrryl, Pyridyl, Pyrimidyl, Pyrazolyl, Imidazolyl, Thiazolyl, Triazyl, Chinaldyl, Chinolinyl, Benzoxazolyl, Benzothiazolyl, Benzothiadiazolyl, Benzimidyzolyl und Isochinolyl.

Als Beispiele für Arylenreste und substituierte Arylenreste X¹ bis X³ seien 1,2-Phenylen, Tetrachlor- und Tetrabrom-1,2-phenylen, 1,2-Naphthylen, 2,3-Naphthylen, 1,8-Naphthylen und 2,2'-Biphenylen aufgeführt, wobei 1,2-Phenylen und Tetrachlor-1,2-phenylen bevorzugt sind.

Acylreste sind zum Beispiel Acetyl, Propionyl, Butyryl und Benzyl.

Geeignete Alkoxycarbonylreste sind beispielsweise Methoxycarbonyl, Ethoxycarbonyl, Propoxycarbonyl, Butoxycarbonyl, Pentoxycarbonyl und Hexoxycarbonyl.

Beispiele für Acylreste sind Methylcarbonyl, Ethylcarbonyl, Propylcarbonyl, Butylcarbonyl und Pentylcarbonyl.

Als Beispiele für Arylsulfonylreste seien insbesondere Phenylsulfonyl und substituiertes Phenylsulfonyl wie p-Tolylsulfonyl, p-Chlorphenylsulfonyl und p-Bromphenylsulfonyl genannt.

Zur Herstellung der Chinophthalonderivate I geht man vorteilhaft von den Chinophthalonen der allgemeinen Formel III 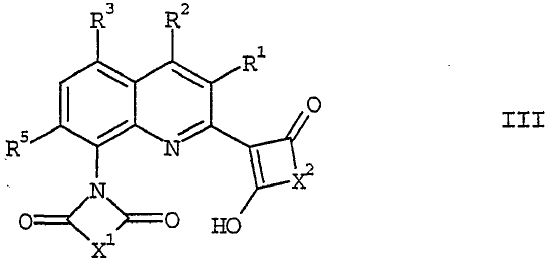 aus.

Durch Umsetzung der Chinophthalone III mit Oleum können die Chinophthalonderivate der Formel I, in der R⁴ -SO₃H bedeutet, erhalten werden.

Zweckmäßigerweise wird für diese Sulfonierung ein Oleum verwendet, das einen Schwefeltrioxidgehalt von 0,1 bis 65 Gew.-%, insbesondere von 5 bis 25 Gew.-%, aufweist.

Die Menge an Oleum ist an sich nicht kritisch. Da das Oleum gleichzeitig als Lösungsmittel dient, sollte mindestens so viel Oleum eingesetzt werden, daß die Lösung rührbar bleibt. Üblicherweise kommen 1 bis 10 g, bevorzugt etwa 3 bis 7 g, Oleum je g Chinophthalon III zum Einsatz.

Die Reaktionstemperatur liegt in der Regel bei 0 bis 200°C, vorzugsweise bei 50 bis 100°C.

Die Reaktionszeit kann 1 bis 20 h betragen. Im allgemeinen ist die regioselektiv erfolgende Sulfonierung in etwa 6 h beendet.

Die Isolierung des Produkts erfolgt üblicherweise nach Hydrolyse des Reaktionsgemisches in Eiswasser durch Abfiltrieren.

Durch weitere Umsetzung mit Aminen oder quartären Ammoniumsalzen können die sulfonierten Chinophthalonderivate I in die entsprechenden Ammoniumsalze I überführt werden (R⁴: -SO₃- N⁺R⁶R⁷R⁸R⁹) , die das Kristallwachstum stärker hemmen als die sulfonierten Chinophthalonderivate I und außerdem die Bildung eines besonders gut dispergierbaren Pigments bewirken.

Als Amine kommen primäre, sekundäre und tertiäre Amine in Betracht. Die sekundären und tertiären Amine können jeweils gleiche oder verschiedene Alkylgruppen enthalten. Geeignet sind beispielsweise auch Fettamine mit linearen, hydrierten oder ungesättigten Alkylresten. Insbesondere kürzere Alkylreste mit bis zu 6 C-Atomen können auch verzweigt sein und/oder bis zu 2 Substituenten aus der bevorzugten Gruppe Hydroxy, Acetyl, Methoxy, Ethoxy, Chlor und Brom tragen.

Beispiele für besonders bevorzugte Amine und Ammoniumsalze sind Stearylamin, Methyldistearylamin, Dimethylstearylamin und Dehydroabietylamin und Dimethyldistearylammoniumsalze.

Die Ammoniumsalzbildung kann auf allgemein übliche Weise in organischen Lösungsmitteln, Wasser oder wäßrig/organischen Medium vorgenommen werden. Geeignete Umsetzungstemperaturen liegen in der Regel bei 20 bis 100°C. Das Produkt kann ebenfalls durch Abfiltrieren isoliert werden.

Das Ammoniumsalz kann jedoch auch erst bei dem erfindungsgemäßen, eine Mahlung und eine Rekristallisation umfassenden Pigmentformierungsverfahren erzeugt werden, indem etwa äquimolare Mengen des Sulfochinophthalonderivates I und des Amins bzw. Ammoniumsalzes bei einem der beiden Formierungsschritte zugegeben werden.

Die Herstellung von Chinophthalonderivaten der Formel I, in der R⁴ -SO₂NR⁶R⁷ bedeutet, kann vorteilhaft durch Sulfochlorierung und anschließende Amidierung erfolgen.

Zur Sulfochlorierung des Chinophthalons III wird üblicherweise Chlorsulfonsäure eingesetzt. Zweckmäßigerweise setzt man das erhaltene Produkt zusätzlich noch mit Thionylchlorid um, um sicherzustellen, daß alle Sulfonsäuregruppen in das Säurechlorid überführt worden sind.

Die Menge an Chlorsulfonsäure ist ebenfalls an sich nicht kritisch. Um jedoch rührbare Lösungen zu erhalten, werden üblicherweise 1 bis 10 g, vorzugsweise etwa 4 g, Chlorsulfonsäure je g Chinophthalon III verwendet.

In der Regel wird diese Reaktion bei 80 bis 180°C, bevorzugt bei 100 bis 130°C, durchgeführt und dauert etwa 1 bis 20 h, vorzugsweise etwa 2 h.

Wird das Produkt nachträglich noch mit Thionylchlorid umgesetzt, so wird das erhaltene Reaktionsgemisch zweckmäßigerweise auf etwa 70 bis 80°C abgekühlt, und dann werden im allgemeinen 0,3 bis 1 g, insbesondere 0,4 bis 0,7 g, Thionylchlorid je g Chinophthalon III zugegeben.

Nach einer weiteren Reaktionszeit von in der Regel 0,5 bis 2 h kann das regioselektiv monosulfochlorierte Produkt nach Hydrolyse in Eiswasser durch Abfiltrieren isoliert werden. Üblicherweise wird es dann ohne vorherige Trocknung der Amidierung zugeführt. Die Amidierung wird mit einem primären oder einem sekundären Amin durchgeführt. Bevorzugt werden die oben aufgeführten Amine für die Umsetzung verwendet.

Vorzugsweise wird die Amidierung in annähernd neutralem, wäßrigem Medium vorgenommen. Um einen pH-Wert von etwa 7 einzuhalten, empfiehlt es sich in Gegenwart eines Puffers, z.B. Natriumacetat, zu arbeiten.

Üblicherweise wird die Amidierung bei einer Temperatur von 0 bis 20°C vorgenommen und dauert etwa 0,5 bis 5 h, insbesondere 1 bis 2 h.

Die Chinophthalonderivate der Formel I, in der R⁴ -CH₂NR⁶R⁷ oder -CH₂R¹⁰ bedeutet, können durch Methylamidierung der Chinophthalone III in einer Tscherniac-Einhorn-Reaktion erhalten werden.

So lassen sich die bevorzugten Phthalimidomethylchinophthalone I durch Umsetzung mit Paraformaldehyd und Phthalimid in konzentrierter Schwefelsäure, die 0,1 bis 10 Gew.-%, bevorzugt 3 Gew.-%, Schwefeltrioxid enthalten kann (0,1 bis 10 gew.-%iges, bevorzugt 3 gew.-%iges, Oleum), herstellen.

In der Regel werden 0,05 bis 0,2 g, vorzugsweise 0,06 bis 0,1 g, Paraformaldehyd und 0,1 bis 0,3 g, bevorzugt 0,2 bis 0,25 g, Phthalimid je g Chinophthalon III eingesetzt.

Die Menge an Schwefelsäure bzw. Oleum ist an sich nicht kritisch. Im allgemeinen kommen 1 bis 10 g, insbesondere etwa 5 g, je g Chinophthalon III zum Einsatz.

Zweckmäßigerweise geht man bei dieser Umsetzung verfahrenstechnisch so vor, daß man Schwefelsäure bzw. Oleum vorlegt, Phthalimid und Paraformaldehyd abwechselnd zugibt und nach einer Reaktionszeit von etwa 0,5 bis 2 h bei 40 bis 60°C das Chinophthalon III zusetzt und das Gemisch dann etwa 2 bis 5 h bei 80 bis 120°C reagieren läßt.

Die Isolierung des Produkts erfolgt üblicherweise nach Hydrolyse des Reaktionsgemisches in Wasser durch Abfiltrieren.

Die Chinophthalonderivate der Formel I, in der R⁴ -COR¹¹ oder C₁-C₄-Alkyl bedeutet, können durch übliche Friedel-Crafts-Acylierung bzw. Friedel-Crafts-Alkylierung des Chinophthalons III erhalten werden. Chinophthalonderivate der Formel I, in der R⁴ -COOH oder -COOR¹¹ bedeutet, sind durch Friedel-Crafts-Acylierung von III und anschließende Umsetzung mit Wasser bzw. Alkoholen erhältlich. Die eine Nitrogruppe als Substituenten R⁴ tragenden Chinophthaloriderivate I sind schließlich durch Nitrierung der Chinophthalone III zugänglich.

Die Chinophthalonderivate I eignen sich hervorragend als Kristallisationsmodifikatoren für organische Pigmente. Sie ermöglichen eine Überführung des Rohpigments in eine für die Anwendung geeignete, feinteilige Pigmentform mit enger Teilchengrößenverteilung.

Besondere Bedeutung haben sie als Kristallisationsmodifikatoren für Chinophthalonpigmente, bevorzugt für Chinophthalonpigmente der eingangs definierten Formel II, besonders bevorzugt für Chinophthalonderivate der Formel II, in der R², R¹⁴ und R¹⁵ Wasserstoff bedeuten, und ganz besonders bevorzugt für C.I. Pigment Yellow 138.

Bei dem erfindungsgemäßen Verfahren zur Überführung von Chinophthalonrohpigmenten in eine feinteilige Pigmentform wird die Formierung des Rohpigments in Gegenwart eines oder mehrerer Chinophthalonderivate I vorgenommen.

Eine bevorzugte Variante des erfindungsgemäßen Verfahrens besteht darin, daß man das bei der Synthese anfallende Rohpigment einer Mahlung, vorzugsweise in Abwesenheit von Mahlhilfsmitteln, unterzieht und das erhaltene Mahlgut anschließend in Gegenwart des Chinophthalonderivates I in einem organischen Lösungsmittel oder einem Gemisch aus organischem Lösungsmittel und Wasser rekristallisiert.

Eine zweite bevorzugte Variante besteht darin, bereits die Mahlung in Gegenwart des Chinophthalonderivates I vorzunehmen und das erhaltene Mahlgut anschließend in einem organischen Lösungsmittel oder einem Gemisch aus organischem Lösungsmittel und Wasser zu rekristallisieren.

Selbstverständlich kann man das Chinophthalonderivat I auch in Teilportionen in unterschiedlichen Phasen des Formierungsprozesses zugeben.

Schließlich kann die Mahlung auch direkt in Gegenwart eines rekristallisierend wirkenden Lösungsmittels, z.B. von C₂-C₄-Alkanolen, Glykolen, Glykolethern und Phthalsäuredialkylestern, insbesondere Phthalsäurediethylester und vor allem Phthalsäuredimethylester, vorgenommen werden. In diesem Fall kann der nachfolgende Rekristallisationsschritt unterbleiben.

Im allgemeinen werden 0,1 bis 10 Gew.-%, bevorzugt 2 bis 5 Gew.-%, Chinophthalonderivat I, bezogen auf das Rohpigment, eingesetzt.

Die Mahlung kann in einer Kugelmühle, Schwingmühle, Planetenmühle oder Rührwerkskugelmühle durchgeführt werden. Geeignete Mahlkörper sind z.B. Eisenkugeln, Silicium/Aluminium/Zirkonoxidperlen, Glasperlen, Achatkugeln und Sandkörner, die Durchmesser im Bereich von 0,1 bis 5 cm aufweisen können.

Vorzugsweise wird so lange gemahlen, bis das Mahlgut eine mittlere Primärteilchengröße < 30 nm aufweist. Dementsprechend beträgt die Mahldauer üblicherweise 10 bis 60 h, insbesondere 30 bis 50 h.

Für die anschließende Rekristallisation kann eine Vielzahl von organischen Lösungsmitteln verwendet werden.

Geeignete Lösungsmittel sind Alkohole insbesondere mit bis zu 10 C-Atomen, Etheralkohole, Ether, Ketone, Carbonsäuren insbesondere mit bis zu 4 C-Atomen, Carbonsäureamide, Carbonsäureester sowie alicyclische und aromatische Kohlenwasserstoffe. Selbstverständlich können auch Mischungen dieser Lösungsmittel eingesetzt werden. Als Beispiele seien im einzelnen genannt:
Methanol, Ethanol, Propanol, Isopropanol, Butanol, Isobutanol, tert.-Butanol, Amylalkohol, Isoamylalkohol, Hexanol, Isohexanol, Heptanol, Octanol, 2-Ethylhexanol, Ethylenglykol, 1,2- und 1,3-Propylenglykol, Cyclohexanol, Methylcyclohexanol, Benzylalkohol und 2-Phenylethanol;
Ethylenglykolmonomethyl-, -ethyl- und -butylether und Diethylenglykolmonomethyl- und -ethylether;
Dipropylether, Diisoproylether, Dibutylether, Diisobutylether, Tetrahydrofuran, Dioxan, Diethylenglykoldimethyl- und -diethylether;
Aceton, Methylethylketon, Methylpropylketon, Methylbutylketon, Diethylketon, Methylisopropylketon, Methylisobutylketon, Cyclopentanon, Cyclohexanon, Methylcyclohexanon, Acetophenon und Propiophenon;
Ameisensäure, Essigsäure, Propionsäure und Buttersäure;
Formamid, N,N-Dimethyl- und N,N-Diethylformamid, N,N-Dimethyl- und N,N-Diethylacetamid, N,N-Dimethyl- und N,N-Diethylpropionsäureamid und N-Methylpyrrolidon;
Phthalsäuredimethylester und Phthalsäurediethylester;
Cyclohexan, Benzol, Toluol, Xylol, Mesitylen, Ethylbenzol, Chlorbenzol, o-Dichlorbenzol, Trichlorbenzol, Naphthalin und Methylnaphthalin.

Bevorzugt werden für die Rekristallisation solche Lösungsmittel verwendet, die sich bei der Aufarbeitung leicht entfernen lassen, z.B. durch Auswaschen mit Wasser, azeotrope Destillation mit Wasser, Wasserdampfdestillation oder durch Trocknen des gesamten Ansatzes (beispielsweise durch Abdestillieren des Lösungsmittels).

Besonders bevorzugt werden solche Lösungsmittel eingesetzt, die einen Siedepunkt ≤ 150°C haben und sich unzersetzt und rückstandsfrei verdampfen lassen, z.B. C₁-C₅-Alkanole, Ketone wie Methylethylketon, Ether wie Tetrahydrofuran und Dioxan und Kohlenwasserstoffe wie Cyclohexan, Benzol, Toluol, Xylol und Chlorbenzol und deren Gemische, wobei Xylol und Toluol ganz besonders bevorzugt sind.

Die Menge an Lösungsmittel ist im allgemeinen nicht kritisch und kann innerhalb weiter Grenzen variiert werden. In der Regel kommen 3 bis 6 g, vorzugsweise 4 bis 5 g, Lösungsmittel je g Mahlgut zum Einsatz.

Üblicherweise nimmt man die Rekristallisation bei einer Temperatur von 25 bis 140°C, insbesondere 60 bis 100°C, vor.

Die Rekristallisation kann unter Dispergieren des Mahlgutes im Lösungsmittel oder auch durch einfaches Verweilenlassen des Mahlgutes im Lösungsmittel erfolgen. Bevorzugt wird die Mischung aus Mahlgut und Lösungsmittel gerührt.

Die Dauer des Rekristallisationsschritts hängt von der Temperatur und dem Lösungsmittel ab. In der Regel ist die Rekristallisation in 1 bis 10 h beendet.

Mithilfe des erfindungsgemäßen Formierungsverfahrens kann die mittlere Teilchengröße der erhaltenen Chinophthalonpigmente gezielt über die Menge an Chinophthalonderivat I variiert und auf den jeweils gewünschten Wert im Bereich von 50 bis 100 nm mit einer Breite der Teilchengrößenverteilung von ± 20 nm eingestellt werden.

Die Bildung besonders feinteiliger Chinophthalonpigmente wird unterstützt, wenn bei der Formierung (bei der Mahlung und/oder der Rekristallisation) weitere Pigmenthilfsmittel zugesetzt werden.

Besonders geeignet sind hierbei Additive auf Basis substituierter Harnstoffe, die durch doppelte Addition von Aminen an Aryldiisocyanate erhältlich ist. Bevorzugt sind dabei die aus der DE-A-29 06 111 bekannten Harnstoffderivate der Formel 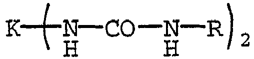 in der K 1,5-Naphthylen oder 4,4'-Diphenylenmethan und R C₁₂-C₁₈-Alkyl, C₁₂-C₁₈-Alkenyl, C₈-C₁₈-Alkoxypropyl oder -(CH₂)₃-O-(C₂H₄O)ₙ-O-R¹ sowie R¹ C₂-C₈-Alkyl oder Phenyl und n 1 bis 4 bedeuten. Als Beispiel für ein bevorzugtes Hilfsmittel sei das durch Addition von 2 mol 3-Octoxypropylenamin an Naphthalin-1,5-diisocyanat erhaltene Produkt genannt.

Die auf diese Weise erhaltenen Pigmentzubereitungen enthalten diese Hilfsmittel als Komponente (C) vorzugsweise in Mengen von 1 bis 10 Gew.-%, bezogen auf das Chinophthalonpigment (A).

Die beim erfindungsgemäßen Formierungsverfahren erhaltenen Chinophthalonpigmente und dementsprechend die ebenfalls erfindungsgemäßen Pigmentzubereitungen, die (A) mindestens ein Chinophthalonpigment und (B) mindestens ein Chinophthalonderivat (B) enthalten, zeichnen sich durch ihre hervorragenden koloristischen und rheologischen Eigenschaften sowie Echtheiten, insbesondere durch hohe Transparenz, hohe Farbstärke und hohen Glanz, leichte Dispergierbarkeit und einwandfreie Überlackier-, Lösungsmittel- und Wetterechtheit, aus. Bei Anwendung in Alkyd/Melamin-Einbrennlacken werden üblicherweise folgende koloristische Eigenschaften erreicht: Hue: 82 bis 86°; Helligkeit L ≥ 80; Chroma C ≥ 90; Transparenz, gemessen in Streu-Delta-E, ≤ 95 bis etwa 40.

Sie eignen sich vorteilhaft zur Einfärbung einer Vielzahl von Anwendungsmedien, z.B. von Kunststoffen, lösungsmittelhaltigen und wasserbasierenden Lacken und aufgrund ihrer Transparenz vor allem auch von Druckfarben, die bei allen gängigen Druckverfahren, z.B. im Offsetdruck, Tiefdruck, Verpackungsdruck, Blechdruck und Textildruck, zum Einsatz kommen können.

Insbesondere können sie aufgrund ihrer Feinteiligkeit auch in Ink-Jet-Tinten, Farbfiltern und elektrophotographischen Tonern und Entwicklern, z.B. Ein-, Zwei- und Mehrkomponentenpulvertonern (auch Ein- oder Zweikomponenten-Entwickler genannt), Magnettonern, Flüssigtonern, Polymerisationstonern und Spezialtonern (vgl. z.B. US-A-5 607 804 und 5 620 820), eingesetzt werden.

Selbstverständlich können sie dabei auch in Kombination mit weiteren Farbmitteln, z.B. mit Mono- und Diazo- und Isoindolinpigmenten wie C.I. Pigment Yellow 12, 13, 14, 17, 139 und 185, zum Einsatz kommen.

### Beispiele

### A) Herstellung erfindungsgemäßer Chinophthalonderivate I

### Beispiel 1

100 g C.I. Pigment Yellow 138, hergestellt gemäß Beispiel 1 der DE-A-17 70 960, wurden portionsweise unter Rühren in 500 g auf etwa 10°C gekühltes 11 gew.-%iges Oleum eingetragen. Das Gemisch wurde dann auf 90°C erhitzt und 6 h bei dieser Temperatur gerührt.

Nach Abkühlen auf 25°C wurde das Reaktionsgemisch in 500 g Wasser gegeben. Das auf diese Weise ausgefällte Produkt wurde abfiltriert, mit Wasser sulfatfrei gewaschen und im Vakuumtrockenschrank bei 90°C getrocknet.

Es wurden 108,8 g des Chinophthalonderivates Ia 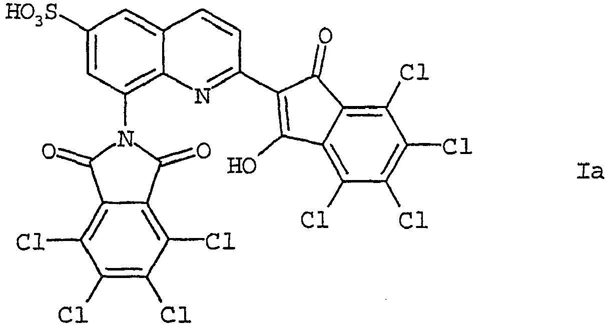 als dünkelgelbes Pulver erhalten, was einer Ausbeute von 98% entspricht.

### Analytische Daten:

Elementaranalyse (Gew.-% ber./gef.):
N: 3,6/3,6; S: 4,1/4,0;
¹H-NMR (D₂SO₄): δ = 8,1; 8,18; 8,47; 8,61 ppm.

### Beispiel 2

Zu einem Gemisch aus 200 g Wasser und 20 g des Chinophthalonderivates Ia aus Beispiel 1 wurden 43,65 g einer 33 gew.-%igen wäßrigen Lösung von Ditalgf ettdimethylammoniumchlorid (Arquad^{®} HC, Akzo Chemicals) unter Rühren zugetropft. Die Suspension wurde dann auf 70°C erhitzt und 30 min bei dieser Temperatur gerührt.

Das erhaltene Produkt wurde abfiltriert, mit Wasser gewaschen und im Vakuumtrockenschrank bei 90°C getrocknet.

Es wurden 27,4 g des Ditalgfettdimethylammoniumsalzes Ib als gelbes Pulver erhalten, was einer Ausbeute von 79% entspricht.

### Beispiel 3

25,5 g Phthalimid und 7,4 g Paraformaldehyd wurden unter Rühren abwechselnd in kleinen Portionen bei 25°C in 560 g 3,6 gew.-%iges Oleum eingetragen. Das Gemisch wurde dann auf 50°C erhitzt und 30 min bei dieser Temperatur gerührt. Nach Zugabe von 100 g eines, wie in Beispiel 1 beschrieben, erhaltenen C.I. Pigment Yellow 138 wurde das Gemisch auf 100°C erhitzt und weitere 3 h bei dieser Temperatur gerührt.

Dann wurde das Reaktionsgemisch in 3500 g Wasser gegeben und 30 min bei 60°C gerührt. Das auf diese Weise ausgefällte Produkt wurde abfiltriert, mit Wasser neutral gewaschen und im Vakuumtrockenschrank bei 90°C getrocknet.

Es wurden 121,2 g des Chinophthalonderivates Ic 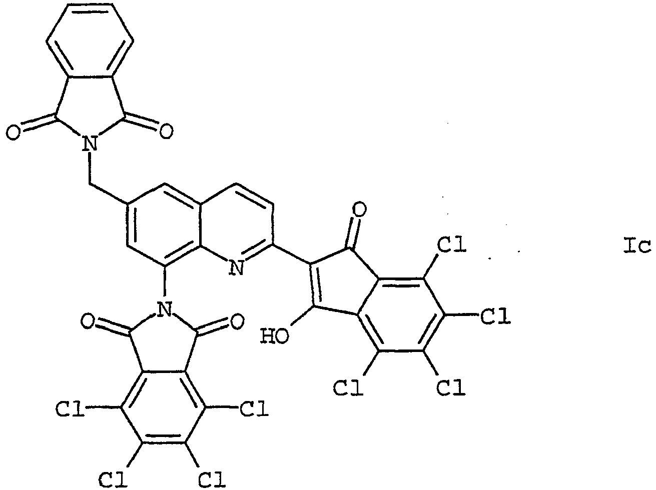 als goldgelbes Pulver erhalten, was einer Ausbeute von 99% entspricht.

### Analytische Daten:

Elementaranalyse (Gew.-% ber./gef.):
N: 4,9/4,9; C1: 33,3/32,4.

### Beispiel 4

a) Ein Gemisch aus 1200 g Chlorsulfonsäure und 200 g eines, wie in Beispiel 1 beschrieben, erhaltenen C.I. Pigment Yellow 138 wurde auf 120°C erhitzt und 2 h bei dieser Temperatur gerührt. Nach Abkühlen auf ≤ 80°C und anschließender Zugabe von 123 g Thionylchlorid wurde weitere 30 min bei 80°C gerührt.
   Nach Abkühlen auf 25°C wurde das Reaktionsgemisch in 3500 g Eis/Wasser-Gemisch gegeben, wobei so viel Eis nachgesetzt wurde, daß die Temperatur nicht über 3°C stieg.
   Nach 15minütigem Nachrühren wurde das ausgefällte Produkt abfiltriert, mit Wasser sulfatfrei gewaschen und ohne Trocknen weiter umgesetzt.
b) Ein Gemisch aus 1000 g Eis/Wasser-Gemisch und 614 g des wasserfeuchten Produkts aus Schritt a) (Gehalt etwa 18,5%) wurde auf < 2°C abgekühlt. Der pH-Wert des Gemischs wurde durch Zugabe von ca. 8 g Natriumcarbonat auf 8,7 eingestellt. Anschließend wurde eine mit 8,15 g Eisessig auf einen pH-Wert von 7 eingestellte Mischung von 29 g Tridecylamin und 100 g Wasser in 30 min unter Rühren zugetropft. Der dabei auf 5,7 fallende pH-Wert wurde durch Zugabe von Natriumcarbonat auf 7 eingestellt.
   Nach einstündigem Nachrühren wurde das Produkt abfiltriert, mit Wasser neutral gewaschen und im Vakuumtrockenschrank bei 90°C getrocknet.

Es wurden 131 g des Chinophthalonderivates Id 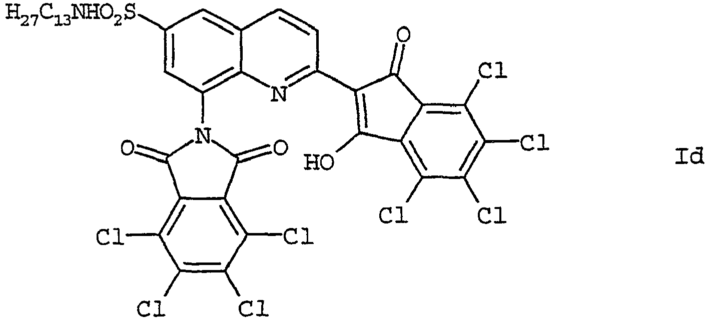 als gelbes Pulver erhalten, was einer Ausbeute von 95% entspricht.

### Analytische Daten:

Elementaranalyse (Gew.-% ber./gef.) :
C: 49,0/49,5; N: 4,4/4,4; Cl: 29,7/30,4.

### B) Herstellung und Anwendung erfindungsgemäßer Pigmentzubereitungen

### Beispiel 5 bis 26

### Variante A

In einer Schwingmühle (11 Volumen) wurden 100 g C.I. Pigment Yellow 138 in Form des gemäß Beispiel 1 der DE-A-17 70 960 erhaltenen Rohpigments mit ca. 1900 g Eisenkugeln (Durchmesser 2 cm) 40 h auf einem Schwingbock geschüttelt.

Ein Gemisch aus 97 g des Mahlgutes, x g des Chinophthalonderivates I, y g des Amins A und 300 g Xylol wurde dann 5 h bei 65°C gerührt. Das Lösungsmittel wurde anschließend bei 120°C im Vakuum abdestilliert, und das Produkt wurde bis zum Erreichen eines Xylolgehaltes < 0,1% getrocknet.

### Variante B

Die Mahlung wurde analog Variante A durchgeführt, jedoch wurden 97 g des Rohpigments in Gegenwart von x g des Chinophthalonderivates I gemahlen.

Das erhaltene Mahlgut wurde analog Variante A unter Zusatz von y g des Amins A in Xylol rekristallisiert.

Bei beiden Varianten wurde das formierte Pigment anschließend pulverisiert und geprüft.

In allen Fällen zeigte das erhaltene, formierte C.I. Pigment Yellow 138 bei elektronenmikroskopischer Untersuchung eine mittlere Teilchengröße (d₅₀-Wert) im Bereich von 50 bis 100 nm mit einer Breite der Teilchengrößenverteilung von ± 20 nm.

Das jeweils erhaltene Pigment war sehr leicht in allen Anwendungsmedien dispergierbar. Bei Einarbeitung in einen Alkyd/Melamin-Einbrennlack wurde ein transparenter, sehr grünstichiger, brillanter Gelbfarbton erhalten.

Einzelheiten zu diesen Versuchen sind Tabelle 1 zu entnehmen.

Dabei wurden folgende Bezeichnungen verwendet:

| | |
|---|---|
| Amin A1: | Ditalgfettdimethylammoniumchlorid (Arquad HC, Akzo Chemicals) |
| Amin A2: | Ditalgfettmethylamin (Armeen^{®} M2HT, Akzo Chemicals) |
| Amin A3: | Dehydroabietylamin (Amine D, Hercules) |

**Tabelle 1**

| Bsp | Variante | x g | Chinophthalonderivat I | y g | Amin A |
|---|---|---|---|---|---|
| 5 | A | 1,5 | Ia | - | - |
| 6 | B | 1,5 | Ia | - | - |
| 7 | A | 3 | Ia | - | - |
| 8 | B | 3 | Ia | - | - |
| 9 | A | 3 | Ib | - | - |
| 10 | B | 3 | Ib | - | - |
| 11 | A | 3 | Ic | - | - |
| 12 | B | 3 | Ic | - | - |
| 13 | A | 3 | Id | - | - |
| 14 | B | 3 | Id | - | - |
| 15 | A | 1,8 | Ia | 1,2 | A1 |
| 16 | B | 1,8 | Ia | 1,2 | A1 |
| 17 | A | 1,8 | Ia | 1,2 | A2 |
| 18 | B | 1,8 | Ia | 1,2 | A2 |
| 19 | A | 1,8 | Ia | 1,2 | A3 |
| 20 | B | 1,8 | Ia | 1,2 | A3 |
| 21 | A | 3 | Ia | 3 | A1 |
| 22 | B | 3 | Ia | 3 | A1 |
| 23 | A | 3 | Ia | 3 | A2 |
| 24 | B | 3 | Ia | 3 | A2 |
| 25 | A | 3 | Ia | 3 | A3 |
| 26 | B | 3 | Ia | 3 | A3 |

### C) Herstellung von Ink-Jet-Tinten

### Beispiel 27

15 g der Pigmentzubereitung aus Beispiel 5, 10 g eines Dispergiermittels D auf Basis eines oxalkylierten Phenols, das in der US-A-4 218 218 als Dispergiermittel 13 beschrieben ist, 5 g 1,2-Propylenglykol und 0,5 g einer 10 gew.-%igen Lösung von 1,2-Benzisothiazolin-3-on in wäßrigem Propylenglykol (Biozid E1) wurden mit vollentsalztem Wasser zum Gesamtgewicht von 100 g aufgefüllt und in einer Mühle angeteigt.

Zur Endeinstellung (4 gew.-%ige Zubereitung) wurden 26,7 g der erhaltenen Mischung mit 3 g Triethylenglykolmonobutylether, 5 g Polyethylenglykol (M_{w} 400 g/mol), 6 g Polytetrahydrofuran 250 (M_{w} 250 g/mol, BASF), 6 g Glycerin, weiteren 0,4 g Biozid E1, 0,5 g eines Netzmittels F auf Basis eines zunächst ethoxylierten und dann propoxylierten 2-(3-Hydroxypropyl)heptamethyltrisiloxans (11 mol EO/5 mol PO) und 1 g Harnstoff versetzt, mit Wasser bis zu einem Gesamtgewicht von 100 g aufgefüllt, gemischt und über ein Sieb mit einer Porengröße von 1 µm filtriert.

### Beispiel 28 und 29

Die Ink-Jet-Tinten der Beispiele 28 und 29 wurden analog Beispiel 27 hergestellt.

Weitere Angaben zu den erhaltenen Ink-Jet-Tinten sind in Tabelle 2 zusammengestellt. Bei den Prozentangaben handelt es sich jeweils um Gew.-%. Biozid E2 bedeutet eine 20 gew.-%ige Lösung von 1,2-Benzisothiazolin-3-on in wäßrigem Ethylenglokyol.

**Tabelle 2**

| | Ink-Jet-Tinte | | |
|---|---|---|---|
| | Bsp. 27 | Bsp. 28 | Bsp. 29 |
| Pigmentzub. Bsp. 5 | 4% | 2% | 4,5% |
| Dispergiermittel D | 2,7% | 1,3% | 3% |
| Polytetrahydrofuran 250 | 6% | 6% | 6% |
| Glycerin | 6% | 6% | 6% |
| 1,2-Propylenglykol | 1,4% | 0,7% | 1,5% |
| Triethylenglykolmonobutylether | 3% | 5% | 5% |
| Polyethylenglykol 400 | 5% | 4% | 4% |
| Harnstoff | 1% | 1% | 1% |
| Netzmittel F | 0,5% | 0,5% | - |
| Biozid E1 | 0,4% | 0,07% | 0,15% |
| Biozid E2 | - | 0,4% | 0,4% |
| vollentsalztes Wasser | 70% | 75,03% | 70,45% |
| Gesamt | 100% | 100% | 100% |

## Patentansprüche

1. Chinophthalonderivate der allgemeinen Formel I 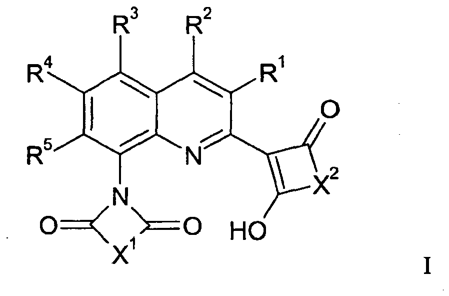 in der die Variablen folgende Bedeutung haben:
R¹, R², R³ und R⁵ unabhängig voneinander Wasserstoff, Halogen oder C₁-C₄-Alkyl;
R⁴ -SO₃H, -SO₃⁻ N⁺R⁶R⁷R⁸R⁹, -SO₂NR⁶R⁷, -CH₂R¹⁰, -COOH, -COON⁺R⁶R⁷R⁸R⁹, -COOR¹¹ oder -NO₂;
R⁶, R⁷, R⁸ und R⁹ unabhängig voneinander Wasserstoff; C₁-C₂₂-Alkyl oder C₂-C₂₂-Alkenyl, dessen Kohlenstoffkette jeweils durch eine oder mehrere Gruppierungen -O-, -S-, -NR¹²-, -CO- oder -SO₂- unterbrochen sein kann und/oder das ein- oder mehrfach durch Hydroxy, Halogen, Aryl, C₁-C₄-Alkoxy und/oder Acetyl substituiert sein kann; C₃-C₈-Cycloalkyl, dessen Kohlenstoffgerüst durch eine oder mehrere Gruppierungen -O-, -S-, -NR¹²- oder -CO- unterbrochen sein kann und/oder das ein- oder mehrfach durch Hydroxy, Halogen, Aryl, C₁-C₄-Alkoxy und/oder Acetyl substituiert sein kann; Dehydroabietyl oder Aryl; R⁶ und R⁷ bzw. R⁶, R⁷ und R⁸ zusammen einen das Stickstoffatom enthaltenden, 5- bis 7-gliedrigen cyclischen Rest, der weitere Heteroatome enthalten kann;
R¹⁰ einen Rest 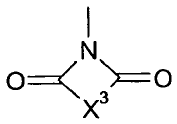
R¹¹ einen der Alkylreste R⁶;
R¹² Wasserstoff oder C₁-C₄-Alkyl;
X¹, X² und X³ unabhängig voneinander Arylen, das durch Halogen, Arylsulfonyl oder -COR¹³ oder -CO-C₆H₄-CO- substituiert sein kann;
R¹³ C₁-C₃-Alkyl oder Phenyl.

2. Chinophthalonderivate der Formel I gemäß Anspruch 1, in der die Variablen folgende Bedeutung haben:
R¹,R², R³ und R⁵ Wasserstoff;
R⁴ -SO₃H, -SO₃⁻ N⁺R⁶R⁷R⁸R⁹, -SO₂NR⁶R⁷ oder -CH₂R¹⁰
R⁶, R⁷, R⁸ und R⁹ unabhängig voneinander Wasserstoff; C₁-C₂₂-Alkyl oder C₂-C₂₂-Alkenyl, dessen Kohlenstoffkette jeweils durch eine oder mehrere Gruppierungen -O-, -S-, -NR¹²-, -CO- oder -SO₂- unterbrochen sein kann und das ein- oder mehrfach durch Hydroxy, Halogen, Aryl, C₁-C₄-Alkoxy und/oder Acetyl substituiert sein kann; Cyclohexyl; Dehydroabietyl oder Aryl;
R¹⁰ einen Rest 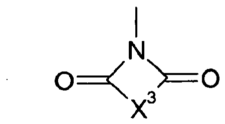
R¹² Wasserstoff oder C₁-C₄-Alkyl;
X¹ und X² 1,2-Phenylen, das bis zu 4 Halogenatome als Substituenten tragen kann;
X³ Arylen, das durch Halogen, Arylsulfonyl oder -COR¹³ oder - CO-C₆H₄-CO- substituiert sein kann;
R¹³ C₁-C₃-Alkyl oder Phenyl.

3. Verwendung von Chinphthalonderivaten der Formel I gemäß Anspruch 1 oder 2 als Kristallisationsmodifikatoren für organische Pigmente.

4. Verfahren zur Überführung von Chinophthalonrohpigmenten in eine feinteilige Pigmentform, **dadurch gekennzeichnet, daß** man die Formierung des Chinophthalonrohpigments in Gegenwart eines oder mehrerer Chinophthalonderivate der Formel I gemäß Anspruch 1 oder 2 vornimmt.

5. Verfahren nach Anspruch 4, **dadurch gekennzeichnet, daß** man das bei der Synthese anfallende Chinophthalonrohpigment einer Mahlung in Abwesenheit von Mahlhilfsmitteln unterzieht und das erhaltene Mahlgut anschließend in Gegenwart des Chinophthalonderivates in einem organischen Lösungsmittel oder einem Gemisch aus organischem Lösungsmittel und Wasser rekristallisiert.

6. Verfahren nach Anspruch 4, **dadurch gekennzeichnet, daß** man das bei der Synthese anfallende Chinophthalonrohpigment einer Mahlung in Gegenwart des Chinophthalonderivates unterzieht und das erhaltene Mahlgut anschließend in einem organischen Lösungsmittel oder einem Gemisch aus organischem Lösungsmittel und Wasser in der Wärme unterzieht.

7. Chinophthalonpigmente der allgemeinen Formel II 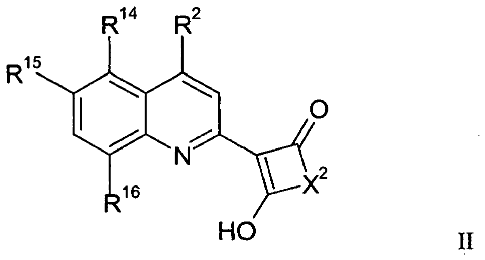 in der die Variablen folgende Bedeutung haben:
R² Wasserstoff, Halogen oder C₁-C₄-Alkyl;
einer der Reste R¹⁴, R¹⁵ und R¹⁶ einen Rest 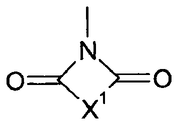 und die anderen Reste R¹⁴ bis R¹⁶ Wasserstoff;
X¹ und X² unabhängig voneinander Arylen, das durch Halogen, Arylsulfonyl oder -COR¹³ oder -CO-C₆H₄-CO- substituiert sein kann;
R¹³ C₁-C₃-Alkyl oder Phenyl,
die isometrische Teilchenform und eine Teilchengröße von 50 bis 200 nm mit einer Breite der Teilchengrößenverteilung von ± 20 nm aufweisen.

8. Pigmentzubereitungen, enthaltend
A) mindestens ein Chinophthalonpigment der allgemeinen Formel II 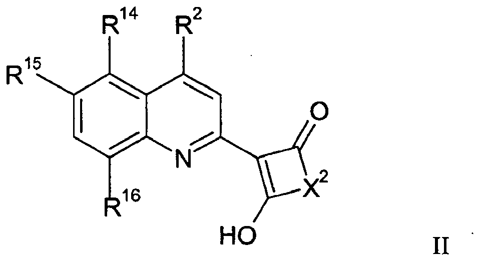 in der die Variablen folgende Bedeutung haben:
R² Wasserstoff, Halogen oder C₁-C₄-Alkyl;
einer der Reste R¹⁴, R¹⁵ und R¹⁶ einen Rest 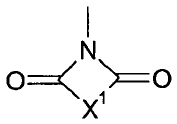 und die anderen Reste R¹⁴ bis R¹⁶ Wasserstoff;
X¹ und X² unabhängig voneinander Arylen, das durch Halogen, Arylsulfonyl oder -COR¹³ oder -CO-C₆H₄-CO- substituiert sein kann;
R¹³ C₁-C₃-Alkyl oder Phenyl,
und
B) mindestens ein Chinophthalonderivat der Formel I gemäß Anspruch 1 oder 2.

9. Pigmentzubereitungen nach Anspruch 8, die, bezogen auf das Chinophthalonpigment (A), 1 bis 10 Gew.-% des Chinophthalonderivates (B) enthalten.

10. Verwendung von gemäß den Ansprüchen 4 bis 6 erhaltenen Chinophthalonpigmenten, Chinophthalonpigmenten gemäß Anspruch 7 und von Pigmentzubereitungen gemäß Anspruch 8 oder 9 zum Einfärben von Kunststoffen, Lacken, Druckfarben, Ink-Jet-Tinten, Farbfiltern und elektrophotographischen Tonern und Entwicklern.

## Claims

1. Quinophthalone derivatives of the general formula I 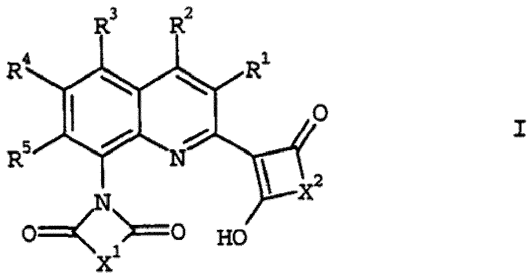 where
R¹, R², R³ and R⁵ are independently hydrogen, halogen or C₁-C₄-alkyl ;
R⁴ is -SO₃H, -SO₃⁻ N⁺R⁶R⁷R⁸R⁹ -SO₂NR⁶R⁷, -CH₂R¹⁰, -COOH, -COO⁻ N⁺R⁶R⁷R⁸R⁹ -COOR¹¹ or -NO₂;
R⁶, R⁷, R⁸ and R⁹ are independently hydrogen; C₁-C₂₂-alkyl or C₂-C₂₂-alkenyl whose carbon chains may be interrupted by one or more moieties selected from the group consisting of -0-, -S-, -NR¹²-, - CO- and -SO₂- and/or which may each be mono- or polysubstituted by hydroxyl, halogen, aryl, C₁-C₄-alkoxy and/or acetyl; C₃-C₈-cycloalkyl whose carbon skeleton may be interrupted by one or more moieties selected from the group consisting of -O-, -S-, -NR¹²- and -CO- and/or which may be mono- or polysubstituted by hydroxyl, halogen, aryl, C₁-C₄-alkoxy and/or acetyl; dehydroabietyl or aryl; R⁶ and R⁷ or R⁶, R⁷ and R⁸ are together a 5- to 7-membered cyclic radical which includes the nitrogen atom and may include further heteroatoms;
R¹⁰ is 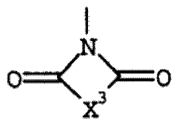
R¹¹ is alkyl R⁶;
R¹² is hydrogen or C₁-C₄-alkyl;
X¹, X² and X³ are independently arylene, which may be substituted by halogen, arylsulfonyl or -COR¹³ or -CO-C₆H₄-CO- ;
R¹³ is C₁-C₃-alkyl or phenyl.

2. Quinophthalone derivatives according to claim 1 of the formula I where
R¹, R², R³ and R⁵ are each hydrogen;
R⁴ is -SO₃H, -SO₃⁻ N⁺R⁶R⁷R⁸R⁹, -SO₂NR⁶R⁷ or -CH₂R¹⁰
R⁶, R⁷, R⁸ and R⁹ are independently hydrogen; C₁-C₂₂-alkyl or C₂-C₂₂-alkenyl whose carbon chains may be interrupted by one or more moieties selected from the group consisting of -0-, -S-, -NR¹²-, - CO- and -SO₂- and which may each be mono- or polysubstituted by hydroxyl, halogen, aryl, C₁-C₄-alkoxy and/or acetyl; cyclohexyl; dehydroabietyl or aryl;
R¹⁰ is 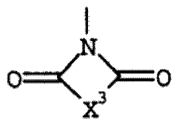
R¹² is hydrogen or C₁-C₄-alkyl;
X¹ and X² are each 1,2-phenylene, which may bear up to 4 halogen atoms as substituents;
X³ is arylene, which may be substituted by halogen, arylsulfonyl or -COR¹³ or -CO-C₆H₄-CO-;
R¹³ is C₁-C₃-alkyl or phenyl.

3. The use of quinophthalone derivatives of the formula I as set forth in claim 1 or 2 as crystallization modifiers for organic pigments.

4. A process for transforming crude quinophthalone pigments into a finely divided pigmentary state, which comprises finishing the crude quinophthalone pigment in the presence of one or more quinophthalone derivatives of the formula I as set forth in claim 1 or 2.

5. The process according to claim 4, wherein the assynthesized crude quinophthalone pigment is ground in the absence of grinding assistants and the millbase obtained is subsequently recrystallized in an organic solvent or a mixture of organic solvent and water in the presence of the quinophthalone derivative.

6. The process according to claim 4, wherein the assynthesized crude quinophthalone pigment is ground in the presence of the quinophthalone derivative and the millbase obtained is subsequently recrystallized in an organic solvent or a mixture of organic solvent and water.

7. Quinophthalone pigments of the general formula II 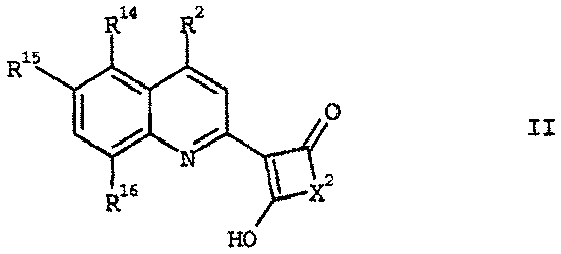 where
R² is hydrogen, I halogen or C₁-C₄-alkyl; one of R¹⁴, R¹⁵ and R¹⁶ is 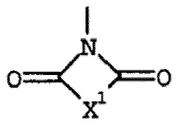 and the others are each hydrogen;
X¹ and X² are independently arylene, which may be substituted by halogen, arylsulfonyl or -COR¹³ or -CO-C₆H₄-CO- ;
R¹³ is C₁-C₃-alkyl or phenyl,
**characterized by** an isometric particle shape and a particle size of from 50 to 200 nm coupled with a width of ± 20 nm for the particle size distribution.

8. Pigment preparations comprising
A) at least one quinophthalone pigment of the general formula II 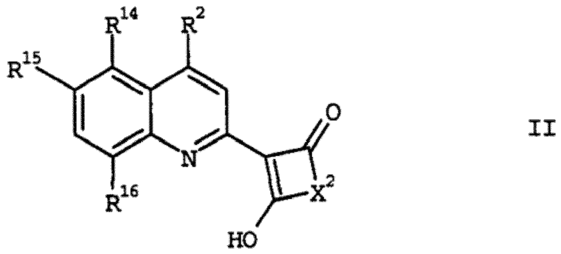 where
R² is hydrogen, halogen or C₁-C₄-alkyl;
one of R¹⁴, R¹⁵ and R¹⁶ is 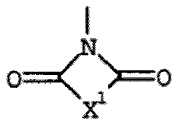 and the others are each hydrogen;
X¹ and X² are independently arylene, which may be substituted by halogen, arylsulfonyl or -COR¹³ or -CO-C₆H₄-CO- ;
R¹³ is C₁-C₃-alkyl or phenyl,
and
B) at least one quinophthalone derivative of the formula I as set forth in claim 1 or 2.

9. Pigment preparations according to claim 8, comprising from 1 to 10% by weight of the quinophthalone derivative (B), based on the quinophthalone pigment (A).

10. The use of quinophthalone pigments obtained according to claims 4 to 6, quinophthalone pigments according to claim 7 and of pigment preparations according to claim 8 or 9 for coloring plastics, paints, printing inks, inkjet inks, color filters and electrophotographic toners and developers.

## Revendications

1. Dérivés de quinophtalone de formule générale I 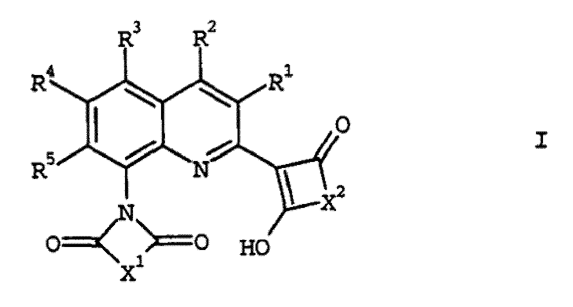 dans laquelle les variables ont la signification suivante :
R¹, R², R³ et R⁵ représentent indépendamment les uns des autres un atome d'hydrogène, un atome d'halogène ou un groupe alkyle en C₁-C₄ ;
R⁴ représente -SO₃H, -SO₃⁻ N⁺R⁶R⁷R⁸R⁹, -SO₂NR⁶R⁷, -CH₂R¹⁰, -COOH, -COO⁻ N⁺R⁶R⁷R⁸R⁹, -COOR¹¹ ou -NO₂ ;
R⁶, R⁷, R⁸ et R⁹ représentent indépendamment les uns des autres un atome d'hydrogène ; un groupe alkyle en C₁-C₂₂ ou alcényle en C₂-C₂₂, dont la chaîne carbonée peut dans chaque cas être interrompue par un ou plusieurs groupements -O-, -S-, -NR¹²-, -CO-ou -SO₂- et/ou qui peut être une ou plusieurs fois substitué par hydroxy, halogéno, aryle, alcoxy en C₁-C₄ et/ou acétyle ; un groupe cycloalkyle en C₃-C₈ dont le squelette carboné peut être interrompu par un ou plusieurs groupements -O-, -S-, -NR¹²- ou -CO- et/ou qui peut être une ou plusieurs fois substitué par hydroxy, halogéno, aryle, alcoxy en C₁-C₄ et/ou acétyle ; un groupe déhydroabiétyle ou aryle ; R⁶ et R⁷ ou respectivement R⁶, R ⁷ et R ⁸ représentent ensemble un radical cyclique à 5 à 7 chaînons contenant l'atome d'azote, qui peut contenir d'autres hétéroatomes ;
R¹⁰ représente un radical 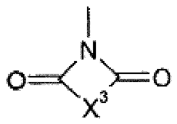
R¹¹ représente l'un des radicaux R⁶ ;
R¹² représente un atome d'hydrogène ou un groupe alkyle en C₁-C₄ ;
X¹, X² et X³ représentent chacun indépendamment un groupe arylène qui peut être substitué par halogéno, arylsulfonyle ou -COR¹³ ou -CO-C₆H₄-CO- ;
R¹³ représente un groupe alkyle en C₁-C₃ ou phényle.

2. Dérivés de quinophtalone de formule I selon la revendication 1, dans lesquels les variables ont la signification suivante :
R¹, R², R³ et R⁵ représentent des atomes d'hydrogène ;
R⁴ représente -SO₃H, -SO₃⁻ N⁺R⁶R⁷R⁸R⁹, -SO₂NR⁶R⁷ ou -CH₂R¹⁰;
R⁶, R⁷, R⁸ et R⁹ représentent indépendamment les uns des autres un atome d'hydrogène ; un groupe alkyle en C₁-C₂₂ ou alcényle en C₂-C₂₂, dont la chaîne carbonée peut dans chaque cas être interrompue par un ou plusieurs groupements -O-, -S-, -NR¹²-, -CO-ou -SO₂- et/ou qui peut être une ou plusieurs fois substitué par hydroxy, halogéno, aryle, alcoxy en C₁-C₄ et/ou acétyle ; un groupe cyclohexyle ; un groupe déhydroabiétyle ou aryle ;
R¹⁰ représente un radical 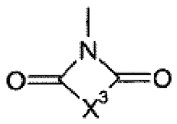
R¹² représente un atome d'hydrogène ou un groupe alkyle en C₁-C₄ ;
X¹ et X² représentent un groupe 1,2-phénylène qui peut porter comme substituants jusqu'à 4 atomes d'halogène ;
X³ représente un groupe arylène qui peut être substitué par halogéno, arylsulfonyle ou -COR¹³ ou -CO-C₆H₄-CO- ;
R¹³ représente un groupe alkyle en C₁-C₃ ou phényle.

3. Utilisation de dérivés de quinophtalone de formule I selon la revendication 1 ou 2, en tant que modificateurs de cristallisation pour pigments organiques.

4. Procédé pour la conversion de pigments quinophtalone bruts en une forme de pigment finement divisée, **caractérisé en ce qu'**on effectue la mise en forme du pigment quinophtalone brut en présence d'un ou de plusieurs dérivés de quinophtalone de formule I selon la revendication 1 ou 2.

5. Procédé selon la revendication 4, **caractérisé en ce qu'**on soumet le pigment quinophtalone brut produit dans la synthèse à un broyage en absence d'adjuvants de broyage et on fait ensuite recristalliser le produit de broyage obtenu, en présence du dérivé de quinophtalone dans un solvant organique ou un mélange de solvant organique et d'eau.

6. Procédé selon la revendication 4, **caractérisé en ce qu'**on soumet le pigment quinophtalone brut produit dans la synthèse à un broyage en présence du dérivé de quinophtalone et on fait ensuite recristalliser le produit de broyage obtenu, dans un solvant organique ou un mélange de solvant organique et d'eau.

7. Pigments quinophtalone de formule générale II 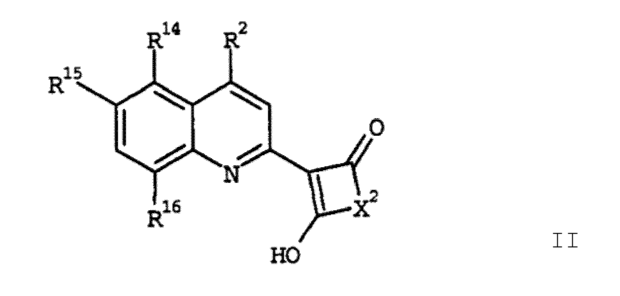 dans laquelle les variables ont la signification suivante :
R² représente un atome d'hydrogène, un atome d'halogène ou un groupe alkyle en C₁-C₄ ;
l'un des radicaux R¹⁴, R¹⁵ et R¹⁶ représente un radical 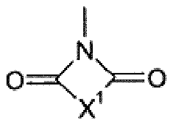
et les autres radicaux R¹⁴ à R¹⁶ représentent des atomes d'hydrogène ;
X¹ et X² représentent indépendamment l'un de l'autre un groupe arylène qui peut être substitué par halogéno, arylsulfonyle ou -COR¹³ ou -CO-C₆H₄-CO- ;
R¹³ représente un groupe alkyle en C₁-C₃ ou phényle,
qui présentent la forme isométrique de particule et une taille de particule de 50 à 200 nm avec une largeur de distribution de tailles de particules de ± 20 nm.

8. Préparation de pigments, contenant
A) au moins un pigment quinophtalone de formule générale II 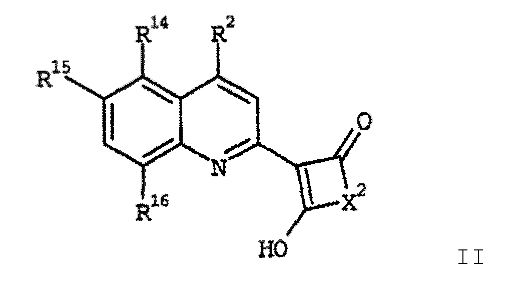 dans laquelle les variables ont la signification suivante :
R² représente un atome d'hydrogène, un atome d'halogène ou un groupe alkyle en C₁-C₄ ;
l'un des radicaux R¹⁴, R¹⁵ et R¹⁶ représente un radical 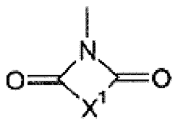 et les autres radicaux R¹⁴ à R¹⁶ représentent des atomes d'hydrogène ;
X¹ et X² représentent indépendamment l'un de l'autre un groupe arylène qui peut être substitué par halogéno, arylsulfonyle ou -COR¹³ ou -CO-C₆H₄-CO- ;
R¹³ représente un groupe alkyle en C₁-C₃ ou phényle,
et
B) au moins un dérivé de quinophtalone de formule I selon la revendication 1 ou 2.

9. Préparations de pigments selon la revendication 8, qui contiennent, par rapport au pigment quinophtalone (A), 1 à 10 % en poids du dérivé de quinophtalone (B).

10. Utilisation des pigments quinophtalone obtenus selon les revendications 4 à 6, des pigments quinophtalone selon la revendication 7 et des préparations de pigments selon la revendication 8 ou 9, pour la coloration de matières plastiques, de peintures, d'encres d'impression, d'encres pour impression par jet d'encre, de filtres colorés et de toners et développeurs électrophotographiques.
